# EUROPEAN PATENT SPECIFICATION

(11) **EP 2 395 974 B1**
(45) Date of publication and mention of the grant of the patent: **07.09.2016**
(21) Application number: 10721627.7
(22) Date of filing: 03.02.2010
(51) Int. Cl.: A61K 9/08, A61K 31/465, A61K 36/00

(54) **A COMPOSITION FOR BUCCAL ABSORPTION OF NICOTINE FOR THE PURPOSE OF SMOKING CESSATION**
ZUSAMMENSETZUNG FÜR NIKOTINABSORPTION IM MUND ZUR RAUCHENTWÖHNUNG
COMPOSITION POUR ABSORPTION BUCCALE DE NICOTINE DANS LE BUT D'UNE DÉSACCOUTUMANCE AU TABAC

(30) Priority: 11.02.2009 CZ 20090080; 09.12.2009 CZ 20090829
(43) Date of publication of application: 21.12.2011
(73) Proprietor: Heglund, a.s., 110 00 Praha 1 (CZ)
(72) Inventor: KOSÍK, Dobromil, 169 00 Praha 6 (CZ); DITTRICH, Milan, 500 11 Hradec Králové (CZ)
(74) Representative: Kratochvil, Vaclav
(86) International application number: PCT/CZ2010/000011
(87) International publication number: WO 2010/091649

(56) References cited:
- WO-A1-2007/093824
- WO-A2-97/38662
- WO-A2-2008/069972
- WO-A2-2009/001085

## Description

### Technology Field:

The invention deals with a composition intended for buccal absorption of nicotine into the systemic circulation and distribution to the central nervous system for the purpose of smoking cessation or nicotine substitution in conditions adverse for smoking.

### Exiting Conditions of Technology:

Harmful effects of smoking have been covered by multiple extensive, statistically assessed studies carried out on large sets of smokers and non-smokers. By smoking of one cigarette pack per day smoker delivers to his/her system circulation a daily dose of 20 to 30 mg (Benowitz NL et al., Clin. Pharmacol. Ther. 44 (1988) 23-28). Nicotine absorbs very quickly, after smoke inhalation it gets to the brain during ten to twenty seconds (Benowitz NL et al., Annu.Rev.Pharmacol.Toxicol. 36 (1996) 597-613). Many proofs exist that brain creates tolerance and addiction to nicotine after its effect on dopaminergic receptors in the brain (Hoffman D et al., Am. J. Health 73 (1983) 1050-1053). It was clearly proved that smoking cessation involves dishabituation to nicotine addiction; therefore it is a nicotinism therapy. It is a usually a long-term and relatively poorly successful process (Cummings KM, Hyland A, Ann. Rev. Pub. Health 26 (2005) 583-599).

Smoking cessation therapies use preparations with various types of nicotine applied in various ways. In order to facilitate application and achieve sufficient nicotine bioavailability the most common absorption method is via buccal mucosa. Smoker endeavours to achieve plasmatic concentration of nicotine within the range from 30 to 50 µg/l (Lawson GM et al., J. Clin. Pharmacol. 38 (1998) 510-516). Slow release of nicotine is not advantageous, key aspect of the addiction is pharmacological response to instantly achieved plasmatic peak of nicotine (Henningfield JE, Keenan RM, J. Consult. Clin. Psychol. 61 (1993) 743-750).

Bioavailability of chewing gums (Shiffman S et al., Addiction 97 (2002) 505-516) containing 2 mg or 4 mg of nicotine is approximately 50% (Benowitz NL, Jacob P III, Savanapridi C, Clin. Pharmacol. Ther. 41 (1987) 467-473). Plasmatic profiles of nicotine concentration over time vary from those during smoking in stability of its levels due to slow and continuous release of nicotine into oral cavity (Henningfield JE et al, Drug Alcohol Depend. 33 (1993) 23-29). Nicotine bioavailability to a large extent depends on pH value in the mouth, no too acid drinks are recommended 15 minutes before and after application of chewing gum (Henningfield JE et al., JAMA 264 (1990) 1560-1564) because salts of in its ionized form feature decreased transmucosal absorption. This application form also causes unpleasant hot sensation in mouth and throat (Henningfield JE et al., CA Cancer J. Clin. 55 (2005) 281-299). Nicotine release rate is rather non-reproducive, some producers recommend not to chew too intensively to avoid overdosing (Pat. US 6,344,222).

Lozenges are similar form of application as chewing gums, nicotine is released by slow dissolving in mouth (Pat. US 4,967,773), much slower than during smoking (Russel MAH et al., Lancet 2 (1985) 1370). Composition with more rapid dissolution of nicotine is patented (Pat. US 6,280,761). To achieve higher bioavailability, pH values from 7 to 9 were recommended, which are controlled by carbonate buffer; composition was sufficiently stable (Pat. US 6,280,761). By increasing the pH value from 7.4 to 8.5 higher ratio of non-ionized form was achieved from 30% to 80% and bioavailability increased 3.8 times (Pat. US 6,110,495). Low efficiency of lozenges was attributed to low nicotine dosage. Therefore in the USA, higher dosages were permitted from 1 mg to 2 mg and 4 mg (Shiffman S et al., Arch. Intern. Med. 162 (2002) 1267-1276). Nicotine release is slow, bioavailability was proved by 25% higher than with chewing gums (Choi JH et al., Nicotine Tob. Res. 5 (2003) 635-644). Another, less common application form, is sublingual tablets. These are very small tablets soluble in water to be placed under the tongue. Nicotine bioavailability in one dosage is similar as with chewing gum (Molander L, Lunell E, Eur. J. Clin. Pharmacol. 56 (2001) 813-819).

Nasal sprays are another application form of nicotine. Preparations of this kind are in the USA available only upon medical prescription (Henningfield JE et al., CA Cancer J. Clin. 55 (2005) 281-299). Dosages by 0.5 mg of nicotine in 50 µl of solvent are applied to each naris. Nicotine is absorbed very rapidly, their advantage is individual dosing of nicotine according to patient's addiction level Schneider NG et al., Clin. Pharmacokinet. 31 (1996) 65-80). First dosages of this preparation very often induce nasal mucous membrane irritation.

Buccal spray, based on alcohol, water or macrogol solution of active substances and flavouring additives with focus on nicotine and its salts, clemastin and steroid hormones sprayed by a pump, is subject to patents WO/1997/038662 and EP 0 904 055. Use of a pump spray for nicotine application into oral cavity has also been described (Pat. US 5,186,925); arrangement of oral spray with nicotine incorporated into microspheres is patent protected as well (Pat. US 5,939,100).

Novel and at present favourite type of products for nicotine application is a so-called electronic cigarette (Pat. EP Appl. 1618803). Nicotine is absorbed by oral cavity and lungs after inhalation. The World Health Organization issued an official statement that they do not recommend this product as an aid to smoking cessation due to lack of information on its safety and efficiency (Anon., Marketers of electronic cigatettes should halt unproved therapy claims. World Health Organization, 2008 - 09 - 19).

Rather problematic may be the influence of not negligible concentration of exhaled nicotine vapours in constrained spaces in transport vehicles on health of non-smokers and especially children whose nicotine detoxifying mechanisms are considerably less efficient than adults'.

Nicotine is a very efficient natural substance belonging to group of alkaloids. It features very rapid start of effect, it influences multiple functions, inter alia heart, blood-vessel and brain. Lethal dose (LD 50) is between 30 and 60 mg (Gosselin RE, Clinical toxicology of commercial products, VI. Ed., Williams & Wilkins, Baltimore 1988, pp 311-313), serious toxic reactions may be experienced, especially by non-smokers, already after dose of 4 to 8 mg, with children this dose is even lower. No antidote efficient against this substance is known.

Transfer of all alkaloids, including nicotine, through biological barriers is easier when their solutions are applied in non-ionized form, i.e. at levels of current acidity exceeding as much as possible the pKa value. The pKa value is a parameter, which expresses the pH value, at which the concentrations of ionized and non-ionized forms of a substance are equal. When applying biologically active substances it is also necessary to consider physiological conditions. Certain compromise between physiologically optimal pH value (isoacid solution) and pH value optimal for permeability through barriers is a so-called euacid solution. This euacid pH value guarantees sufficient bioavailability at pH value suitable also for biological environment as well as for applied substance. Biological availability is parameter, which expresses ratio from the total amount of applied substance, which gets into system circulation or to the target tissue or structure.

In case of many substances the euacid pH value is achieved by combining suitable ratio of a substance in form of the acid or base and its salt. In case of nicotine according to the invention it involves a combination of nicotine base with its salt formed by addition of suitable amount of acid.

In addition to increasing the ratio of non-ionized form as a means to increase the substances bioavailability, specific option in case of buccal application of substances is to decrease barrier function of mucin. Mucin is a high-molecular glycoprotein featuring high viscosity of water solutions. Its viscosity can be reduced by substances called mucolytic agents. From today's perspective, already a classic group of mucolytic agents is compounds containing in their molecule a sulfhydryl group, which causes reduction of crosslinking of mucin molecule by mechanism of reduction of disulphide groups. Reaction is accompanied by significant decrease of solution viscosity, which is exploited in the sphere of solving symptoms of pulmonary diseases and respiratory system diseases of various seriousness. This is the effect of amino acids cysteine, methionine and their esters and ethers (Takatsuka S et al., Int. J. Pharm. 349 (2008) 94-100).

There are many other, non-sulphydryl group based mucolytic agents, acting by various mechanisms. Various enzymes have depolymerization effect on mucin (Rubin B K, Respiratory Care, 52 (2007) 859-865). Traditionally used in practice are solutions of electrolytes, such as ammonium chloride and sodium chloride, causing the mucin's macromolecules covering layer to dehydrate partially, thus decreasing viscosity of its solution. Use of processed sea water as a mucolytic agent in aerosol form is patent protected (Pat. US 7097852). Combinations of electrolytes with hypertonic solutions of saccharides (honey, figs, etc.) or polyhydric alcohols (mannitol, sorbitol, xylitol, pentaerythritol) are used in traditional and alternative medicine mainly in paediatrics.

In therapeutic practice, many phytocomplexes obtained by extraction from various parts of plant are widely used. Sustained tradition attained extractions from blossoms or roots of plant Primula veris or Primula elatior containing triterpene saponines and phenolic glycosides (Anon.: Assessment report on Primula veris, Primula elatior (L.), radix. EMEA(HPMC/144474/2006). Saponins have significant effect in water solution by surface activity mechanism. Use of plant saponins and sapogenins for decreasing the human sputum viscosity as part of its microbiological analysis is patent protected (Pat. US 4053363). Similar effect is shown by triterpene saponins contained in leaves of Hedera helix (Kraft K, Zeitschr. Phytotherap. 25 (2004) 179-181; Fazio S et al., Phytomedicine 16 (2009) 17-24). Essential oils of Eucalyptus globulus contain mainly 1,8-cineol (eucalyptol) and also various substances, such as terpenes, sesquiterpenes and sesquiterpenols acting using various mechanisms, inter alia also mucolytic one (Tibballs J, Med. J. Aust. 163 (1995) 177-180). Also roots of Liquiritia glabra offer a complex of surface active substances, which after separation of undesired glycyrrhizin has mucolytic effects (Guslandi M, Int. J. Clin. Parmacol. Ther. Toxicol., 23 (1985) 398-402). Combination of substances contained in Lichen islandicus and Althea officinalis together with local anaesthetic is part of WO/2006/032364. Blossoms of species Tussilago farfara and genus Verbascum contain a complex of substances, in particular flavonoids and saponins types, which inter alia have also mucolytic effect (Newall C A, Anderson L A, Phillipson J D, Herbal Medicines, Pharmaceutical Press, London 1996). There are many other phytocomplexes used in practice with declared mucolytic effect, such as Euphrasia, Origanum, Pimpinella, Thymus, Trifolium, etc.

Nicotine is a relatively stable substance; exposed to air it decomposes by oxidation, decomposition is accelerated by light. Reaction is catalyzed by strong acceptors of electrons, inhibited by substances capturing radicals. During decomposition reaction are produced mainly N-oxides of alkylpyrrolidines (Suffredini HB et al., Anal. Letters 38 (2005) 1587-1599) and in smaller extent also pyrrolidinones (Beckwith ALJ et al., Austral. J. Chem. 36 (1983) 719-739). In nicotine adsorbed on various celluloses was found also kotinin, methanon-(1-methyl-3-pyrrolidinyl)-3 -pyridinyl, and other degradation products (Mihranyan A, Andersson S-B, Ek R, Eur. J. Pharm. Sci. 22(2004) 279-286). One of the methods to stabilize nicotine is its adsorption on cellulose from algae, bacteria or fungi (WO/2005/023227). Nicotine is metabolized especially in liver, tens of metabolites were found (Moyer TP et al., Clin. Chem. 48 (2002) 1460-1471). In order to stabilize nicotine addition of water soluble antioxidants is necessary.

### Summary of the invention

Composition intended for buccal absorption of nicotine into the system circulation and distribution to the central nervous system for the purpose of smoking cessation or nicotine substitution in conditions adverse for smoking according to the invention is characterized by the fact that it contains nicotine solution in form of a base and/or its salt with organic acid in concentration 0.10 to 4.00% by weight, most preferably 0.20 to 1.00% by weight, further it contains substances with mucolytic effect selected from the group formed by acetylcysteine in the amount of 3.0 to 30.0% by weight and/or phytocomplexes with mucolytic effect obtained by extraction from plants selected from the group of Primula veris, Primula elatior, Hedera helix, Eucalyptus globulus or Liquiritia glabra in concentration from 0.50 to 15.00% by weight.

Composition may further contain 0.05 to 1.50% by weight of phytocomplexes with antioxidizing effect selected from the group of flavones, flavonols, flavanones, flavanonols, isoflavones, neoflavonoids, anthocyanidins, proanthocyanidins and their oligomers and polymers obtained by extraction from plants Camellia sinensis, Curcuma longa, Rosmarinus officinalis, Sylibium marianum, Vitis vinifera, Ginkgo biloba, Euterpe oleracea, Vaccinium myrtillus, Morinda citrifolia, Malpighia glabra.

Composition advantageously further contains buffer or substance adjusting current acidity in the range of pH value from 6.0 to 9.5, more preferably from 7.0 to 9.0, most preferably 8.5.

As a solvent, the composition contains water in concentration 50 to 99% by weight or its mixture with polyhydric alcohol, such as glycerol, propylene glycol, and/or saccharide selected from the group of mannitol, xylitol, glucose, fructose, saccharose in concentration from 2 to 50% by weight.

Composition also advantageously contains at least one component from the group of substances adjusting taste perceptions, substances adjusting olfactory perceptions, antimicrobials, sequestrants, antioxidants, solubilizing agents and agents facilitating wettability, substances improving physical parameters of applied compositions, such as viscosity and surface tension, and substances improving condition of oral cavity surfaces, i.e. mucosae or teeth.

Composition according to the invention is intended for application of nicotine into oral cavity in form of colloid or heterogene aerodispersion by spray device with dispenser with individual dosages volume in the range from 50 µl to 2000 µl, preferably from 150 µl to 500 µl.

Single application dosis contains 0.05 to 3.00 mg, more preferably 0.10 mg to 2.00 mg, most preferably 0.25 mg to 1.00 mg of nicotine.

### Detailed Description of the Preferred Embodiments

Apart from nicotine and its salt as an active substance, the composition contains mucolytic agent, such as acetylcysteine and/or phytocomplexes with mucolytic effect obtained by extraction from plants as a nicotine bioavailability enhancer. It may further contain antioxidant of natural plant origin as a substance stabilizing the nicotine against oxidation and water as a solvent. Other ingredients, which may be advantageously exploited in the composition, are stabilizers of various type, i.e. antimicrobials, sequestrants, free radical quenching agents; substances adjusting organoleptic properties may also be used. Compositions according to the invention may also include substances improving physical parameters of applied compositions, such as viscosity and surface tension, and substances improving conditions of oral cavity surfaces, i.e. mucosae or teeth.

Nicotine is very efficient alkaloid readily soluble in water to strongly alkaline solution. As its tolerance by the buccal mucosa is good and absorption through buccal mucosa into the system circulation is sufficient, it is suitable that pH value of nicotine solutions is lower than 9.0 and higher than 7.0. It is convenient to reduce the pH value of water solutions of nicotine using their mixture with salts of acids, such as citric acid, malic acid, tartaric acid, lactic acid, glycolic acid, phosphoric acid, etc. Concentration of nicotine in solution is determined by volume of applied dosages; it can be from 0.01% to 8.00%, more preferably from 0.10% to 4.00%, most preferably from 0.20% to 1.00%. Individual applied dosages of nicotine may be in the range from 0.05 mg to 3.00 mg, more preferably from 0.10 mg to 2.00 mg, most preferably from 0.25 mg to 1.00 mg.

Mucolytic agents are strongly hydrophilic substances reducing viscosity of mucin, such as substances of mucoprotein type. Reduced viscosity of mucoproteins relates to faster diffusion of nicotine molecules to the surface of mucosa barrier, thus leading to faster and more intense absorption of this substance. Acetylcysteine is a strongly hydrophilic substance. Carboxyl group may interact with basic nitrogen of pyrrolidine cycle of nicotine. It has antioxidizing effect, sulfhydryl group may be a donor of electrons. It is a significant mucolytic agent, it reduces viscosity of water solutions of mucoproteins by interaction with disulphide bonds.

Mucolytic agents of natural plant origin suitable for increase of nicotine bioavailability in the composition are identical with those traditionally used as expectorant substances facilitating expulsion of sputum, especially in paediatrics. Phytocomplexes are therefore used, obtained by extraction from plant drugs, such as water or alcohol extracts from blossoms or roots of plant Primula veris or Primula elatior containing triterpene saponins and phenolic glycosides. Triterpene saponins contained in leaves of Hedera helix have similar effects. Composition according to the invention also may suitably contain essential oil obtained from leaves of Eucalyptus globulus, which contains especially 1,8-cineol (eucalyptol) and also various substances such as terpenes, sesquiterpenes and sesquiterpenols. Also phytocomplex from Liquiritia glabra obtained by extraction from roots offers surface active substances, which after separation of undesired glycyrrhizin have mucolytic effects. Another suitable ingredient in the composition is a combination of substances contained in Lichen islandicus, Althea officinalis, and also substances contained in species Tussilago farfara and genus Verbascum. There are many other phytocomplexes, which can be used with required mucolytic effect, such as Euphrasia, Origanum, Pimpinella, Thymus, Trifolium, etc. Phytocomplexes with mucolytic effect may be used as an ingredient of the composition in concentration from 0.5% by weight to 15% by weight, more preferably in concentration from 0.5% by weight to 5% by weight, most preferably in concentration from 1 % by weight to 2% by weight.

Antioxidants are substances, which in given composition are significant for nicotine stabilization in its solution against oxidizing reactions, also they have positive effect on buccal mucosa. As part of the compositions according to the technical solution are suitable substances of natural plant origin or their mixtures soluble in water, such as ascorbic acid and its salts, various flavonoids, such as flavones, flavonols, flavanones, flavanonols, isoflavones, neoflavonoids, anthocyanidins, proanthocyanidins and their oligomers and polymers. From phytocomplexes obtained by extraction of plant materials containing substances of given category we may mention the extract from leaves of plant Camellia sinensis containing catechins from the group of flavanols, further the extract from Silybium marianum containing complex silymarin, extract from Curcuma longa containing complex curcumine, also extract from Rosmarinus officinalis containing rosemary acid, extract from Vitis vinifera containing proanthocyanidins and other polyphenolic substances of various type, extract from leaves of plant Ginkgo biloba containing flavonoids, fruits of Vaccinium myrtillus containing anthocyanidins, just like Euterpe oleracea. Apart from epicatechiris contained in green tea, flavanol quercetin and polyphenol resveratrol are the most significant in common usage as part of food. Extract from French coastal pine tree Pinus pinaster ssp. atlantica contains a mixture of many bioflavonoids type catechins, oligomeric procyanidins and phenolic fruit acids with antioxidizing potential. Significant sources of ascorbic acid and its salts are extracts from plants Morinda citrifolia and Malpighia glabra. Concentration of phytocomplexes obtained by extraction from plants containing various antioxidants in the composition is in range from 0.05% by weight to 1.5% by weight, more preferably from 0.25% by weight to 1.5% by weight, most preferably from 0.50% by weight to 1.0% by weight. Water in the composition acts as a solvent for individual ingredients. Its ratio in the composition according to the technical solution of the invention is in range from 50% to 99 %, more preferably from 65% to 90%, most preferably from 75% to 95%.

Ingredient used to adjust current acidity may be an acid or hydroxide, possibly amine or amino acid creating partial salts with nicotine or more advantageously buffer system acetate, citric, trolamin, tromethamol, carbonate, etc. Also suitable are dicarboxylic amino acids individual or in combination, such as glutamic acid or aspartic acid, or basic amino acids, such as lysine or arginine. With respect to nicotine stability and bioavailability it is advantageous to adjust pH value of solutions in range from 6.0 to 9.5, more preferably from 7.0 to 9.0, most preferably to pH 8.5.

Other ingredients, which can be advantageously exploited in the composition discussed by the invention, are preservation additives. As those suitable ones appear to be acids and salts of sorbic acid or benzoic acid. Other antimicrobic substances suitable for given purpose may include esters of parahydroxybenzoic acid, or parabens, especially methylparaben. Also suitable are various essentials oils, such as clove oil, thyme oil, wild thyme oil, eucalyptus oil, or extracts from these plants in semipolar solvents. Antimicrobial effect can be achieved also by propylene glycol or ethanol, which can be used also as extraction agents for plant materials. As preserving agents are suitable alcoholic or propylene glycol liquid extracts from plants, such as St. John's wort, Iceland lichen, Neem, sage, willow bark, etc.

Substances adjusting taste perceptions include mainly sweeteners. Most commonly used are various saccharides and inositols, such as glycerol, propylene glycol, saccharose, glucose, fructose, sorbitol, mannitol, xylitol, bee honey, etc. Significant group, which can be used for given purposes, is artificial non-nutritive sweeteners, such as saccharin salt, acesulfame salt, aspartame, neohesperidine dihydrochalcon, sucralose, extract from liquorice, extract from stevia, etc. Olfactory perceptions may be modified by essential oils, such as peppermint oil, spearmint oil, anise oil, fennel oil, eucalyptus oil, coriander oil, cumin oil, cinnamon oil, clove oil, thyme oil, wild thyme oil, dropwort oil, juniper oil, marjoram oil, ginger oil, etc.

Compositions according to the invention may further also include other ingredients adjusting their physical parameters in a positive sense. Viscosity and surface tension of aqueous solutions of substances may be reduced by adding ethanol or propylene glycol, surfactants may also be used. As substances positively affecting conditions of surfaces in oral cavity, i.e. mucosae and teeth, may include wide range of vitamins and minerals, such the complex of vitamins B and their salts, vitamin C and its salts, esters and glycosides, vitamin H, coenzyme Q10, salts of zinc, copper, selenium, magnesium, calcium, manganese, etc. As antiinflammatory agents may be used alpha-bisabolol, allantoin, extract from St. John's wort, marigold, chamomile, etc.

Composition according to the invention is intended for application to oral cavity, onto the surface of buccal mucosa, gingival mucosa, hard palate and tongue by spray, i.e. by heterogenous or colloid dispersion of the composition in gas. As a generator of aerodispersion in the air may be either a pump or spraying may also be provided by flow of gas propeller from a pressure vessel. Propelling gas may advantageously be nitrogen or other chemically relatively inert gas, or mixture of alkanes, such as butane, isobutane or propane. Size of dispersed particles of mist or aerosol may be in range from 20 nm to 100 µm, preferably from 500 nm to 5 µm. For application is suitable, if the generator of aerodispersion is equipped with dispenser device. Volumes of liquid applied in single doses may be in range from 50 µl to 2000 µl, preferably from 150 µl to 500 µl. Listed parameters are indicative only, they are not a subject of the technical solution.

### Examples of Preferred Embodiments

### Example 1, 2, 3:

| Ingredient | Composition (% by weight) | | |
|---|---|---|---|
| | **Sample No. 1** | **Sample No. 2** | **Sample No. 3** |
| Nicotine | 1.00 | 2.00 | 3.00 |
| Citric acid | 0.95 | 1.55 | 2.10 |
| Acetylcysteine | 25.00 | 15.00 | 5.00 |
| Sodium Ascorbyl Phosphate | - | 3.00 | 5.00 |
| Potassium sorbate | 0.10 | 0.10 | 0.10 |
| Sodium benzoate | 0.10 | 0.10 | 0.10 |
| Sucralose | 0.07 | 0.08 | 0.10 |
| Glycerine | 5.00 | 10.00 | 15.00 |
| Grape seed extract | 1.50 | 0.75 | - |
| Perfume | 0.10 | 0.20 | 0.35 |
| Hydrogenated pegylated castor oil | 0.12 | 0.12 | 0.12 |
| Sodium hydroxide | q.s. pH 7.0 - 9.0 | q.s. pH 7.0 - 9.0 | q.s. pH 7.0 - 9.0 |
| Water | up to 100.00 g | up to 100.00 g | up to 100.00 g |

### Example 4, 5, 6, 7:

| Ingredient | Composition (% by weight) | | | |
|---|---|---|---|---|
| | Sample No. 4 | Sample No. 5 | Sample No. 6 | Sample No. 7 |
| Nicotine | 1.00 | 0.2 | 2 | 1 |
| Citric acid | 0.95 | 1.55 | 1.55 | 2.10 |
| Primula veris extract | 2.00 | - | - | - |
| Hedera helix extract | - | 3.00 | - | - |
| Liquiritia glabra extract | - | - | 10.00 | - |
| Tussilago farfara extract | - | - | - | 1.00 |
| Camellia sinensis extract | 1.00 | - | - | - |
| Rosmarinus officinalis extract | - | 0.50 | - | - |
| Equisetum arvense extract | - | - | 0.30 | - |
| Ginkgo biloba extract | - | - | 1.00 | - |
| Vitis vinifera extract | - | - | - | 0.10 |
| Potassium sorbate | 0.10 | 0.10 | 0.1 | 0.10 |
| Sodium benzoate | 0.10 | 0.10 | 0.1 | 0.10 |
| Sucralose | 0.07 | 0.08 | 0.07 | 0.10 |
| Glycerine | 5.00 | 10.00 | 10 | 15.00 |
| | | | | |
| Aroma | 0.10 | 0.20 | 0.3 | 0.35 |
| Hydrogenated pegylated castor oil | 0.12 | 0.12 | 0.12 | 0.12 |
| Sodium hydroxide | q.s. pH 7.0-9.0 | q.s. pH 7.0-9.0 | q.s. pH 7.0-9.0 | q.s. pH 7.0-9.0 |
| Water | up to 100.00 g | up to 100.00 g | up to 100.00 g | up to 100.00 g |

Preparation procedure: Individual ingredients, gradually added, are dissolved in water. Solution is filtered if necessary and filled into pressure vessels with dispenser valve or into closed containers with dispenser valve driven by manually operated pump.

### Industrial Applicability

Composition according to the invention may be produced by pharmaceutical industry. Preparation is suitable for transmucosal absorption of nicotine for the purpose of smoking cessation.

## Claims

1. Composition for buccal absorption of nicotine into the system circulation and distribution to the central nervous system applicable in form of colloid or heterogene aerodispersion by spray device for the purpose of smoking cessation or nicotine substitution in conditions adverse for smoking according to the invention is **characterized by** the fact that it contains nicotine solution in form of base and/or its salt with organic acid in concentration 0.10 to 4.00% by weight, most preferably 0.20 to 1.00% by weight, further it contains substances with mucolytic effect selected from the group formed by acetylcysteine in the amount of 3.0 to 30.0% by weight and/or phytocomplexes with mucolytic effect obtained by extraction from plants selected from the group Primula veris, Primula elatior, Hedera helix, Eucalyptus globulus or Liquiritia glabra in concentration from 0.50 to 15.00% by weight, 0.05 to 1.50% by weight of Phytocomplexes with antioxidizing effect selected from the group of flavones, flavonols, flavanones, flavanonols, isoflavones, neoflavonoids, anthocyanidins, proanthocyanidins and their oligomers and polymers obtained by extraction from plants Camellia sinensis, Curcuma longa, Rosmarinus officinalis, Sylibium marianum, Vitis vinifera, Ginkgo biloba, Euterpe oleracea, Vaccinium myrtillus, Morinda citrifolia, Malpighia glabra and further contains buffer or substance adjusting current acidity in the range of pH value from 7.0 to 9.0.

2. Composition according to claims 1 **characterized by** the fact that as a solvent it contains water in concentration 50 to 99% by weight or its mixture with polyhydric alcohol, such as glycerol, propylene glycol, and/or saccharide selected from the group mannitol, xylitol, glucose, fructose, saccharose in concentration from 2 to 50% by weight.

3. Composition according to claims 1 to 2 **characterized by** the fact that it contains at least one component from the group of substances adjusting taste perceptions, substances adjusting olfactory perceptions, antimicrobials, sequestrants, antioxidants, solubilizing agents and agents facilitating wettability, substances improving physical parameters of applied compositions, such as viscosity and surface tension, and substances improving condition of oral cavity surfaces, i.e. mucosae or teeth.

4. Composition according to claims 1 to 3 **characterized by** the fact that a single application dosage contains 0.10 mg to 2.00 mg, most preferably 0.25 mg to 1.00 mg of nicotine.

## Patentansprüche

1. Komposition der bukkalen Aufnahme von Nikotin in den systematischen Kreislauf und die Verteilung des zentralen Nervensystems, die in Form von Kolloid oder durch heterogene Aerodispersion durch Bestäuber für die Einstellung von Rauchen oder Nikotinersatz in für das Rauchen ungünstige Bedingungen und durch die Erfindung kann sie dadurch charakterisiert werden, dass sie eine Nikotinflüssigkeit beinhaltet und zwar in Form einer Basis und/oder seiner Salze mit organischen Säuren mit einer Konzentration von 0.10 bis 4.00 % nach Gewicht, bevorzugt bei 0.20 bis 1.00 % nach Gewicht. Des Weiteren umfasst es Substanzen mit schleimlösendem Effekt, die von einer Gruppe ausgewählt wurden, die durch Acetylcysteine geformt wurden und zwar in einer Menge von 3.0 bis 30.0% mach Gewicht und/oder Phytocomplexe mit schleimlösendem Effekt, das durch die Extraktion von Pflanzen aus folgenden Gruppen gewonnen werden soll: Primula veris, Primula elatior, Hedera Helix, Eucalyptus globulus oder Liquirtia glabra in einer Konzentration von 0.50 bis 15.00% nach Gewicht und 0.05 bis 1.50% je nach Gewicht der Phytokomplexe mit einem antioxidisierendem Effekt, der von einer Gruppe von Flavonen, Flavonolen, Flavononen, Flavononolen, Isoflavonen, Neflavonoiden, Anthocyanidinen, Proanthocyaniden und ihren Ologorene und Polymeren, die durch die Extraktion folgender Pflanzen gewonnen werden konnten: Camellia Sinensis, Curcuma longa, Rosmarinus, officinalis, Sylibium marianum, Vitis vinifera, Gingko biloba, Euterpe oleracea, Vaccinum myrtillus, Morinda citrifolia, Malpighia glabra und weitere Zwischenspeicher oder Substanzen beinhalten, die sich an die derzeitige Säuren mit einem pH-Wert von 7.0 bis 9.0 beinhalten.

2. Die Zusammensetzung nach Anspruch 1 ist dadurch charakterisiert, dass es als lösendes Mittel Wasser in einer Konzentration von 50 bis 99 % nach Gewicht oder ihrer Mischung mit polyhydridem Alkohol wie z.B. Glycerol, propylenes Glycol und/oder Saccharide, die aus der Gruppe der Mannitole, Xylitole, Glukose, Fruktose, Saccharose in einer Konzentration von 2 bis 50% nach Gewicht ausgewählt werden.

3. Die Zusammensetzung nach den Ansprüchen 1 und 2 wird dadurch charakterisiert, dass es mindestens eine Komponente der Substanzgruppe, die sich an die Geschmackswahrnehmung anpassen, Substanzen, die sich an die Geruchswahrnehmungen anpassen sowie Antimikrobielle, Komplexbildner, Antioxidationsmittel, lösende Mittel, und Mittel zur Vereinfachung von Benetzbarkeit, Substanzen zur Verbesserung der physikalischen Parameter angewandter Zusammensetzungen wie Zähflüssigkeit und Oberflächendruck und Substanzen, welche die Bedingungen in der Mundhöhle insbesondere der Schleimhaut und der Zähne verbessern.

4. Die Zusammensetzung nach den Ansprüchen 1 bis 3 werden durch eine einzelne Anwendung der Dosierung von 0.10 mg bis 2.00 mg, bevorzugt 0.25 mg bis 1.00 mg Nikotin beschrieben.

## Revendications

1. La composition pour l'absorption buccale de la nicotine dans le système circulatoire et la distribution dans le système nerveux central applicable sous forme de l'aérodispersion colloïde ou heterogène par un dispositif de pulvérisation en vue de la cessation du tabagisme ou la substitution de la nicotine dans les conditions défavorables au tabagisme, se caractérise, selon l'invention, par le contenu de la solution de nicotine sous forme d'une base et/ou son sel avec de l'acide organique dans la concentration de 0.10 à 4.00 % en poids, de préférence de 0.20 à 1.00 % en poids. A part cela, la composition contient les substances dotées de l'effet mucolytique sélectionnées du groupe formé par l'acétylcystéine dans la quantité de 3.0 à 30.0 % en poids, et/ou les phytocomplexes d'un effet mucolytique obtenu par l'extraction des plantes sélectionnées du groupe *Primula veris, Primura elatior, Hedera helix, Eucalyptus globulus* ou *Liquiritia glabra* dans la concentration de 0.50 à 15.00 % en poids, 0.05 à 1.50 % en poids des phytocomplexes avec un effet antioxydant sélectionnés du groupe des flavones, flavonols, flavanones, flavanonols, isoflavones, néoflavonoïdes, anthocyanidines, proanthocyanidines et leurs oligomères et polymères obtenus par l'extraction des plantes *Camellia sinensis, Curcuma longa, Rosmarinus officinalis, Sylibium marianum, Vitis vinifera, Ginkgo biloba, Euterpe oleracea, Vaccinium myrtillus, Morinda citrifolia, Malpighia glabra* ; en contenant aussi un tampon ou bien une substance ajustant l'acidité actuelle sur l'échelle de la valeur de pH de 7.0 à 9.0.

2. La composition selon la demande no. 1 contient, en tant que solvant, de l'eau dans la concentration de 50 à 99 % en poids, ou son mélange avec de l'alcool polyhydrique comme le glycérol, le propylène glycol, et/ou le saccharide sélectionnés du groupe mannitol, xylitol, glucose, fructose, saccharose dans la concentration de 2 à 50 % en poids.

3. La composition selon les demandes 1 à 2 se **caractérise par** le contenu d'au moins un composant du groupe des substances ajustant la perception du goût, les substances ajustant la perception olfactive, les antimicrobiens, séquestrants, antioxydants, agents solubilisants et les agents facilitant la mouillabilité, les substances améliorant les paramètres physiques des compositions appliquées comme la viscosité et la tension de surface, et les substances améliorant la condition de la surface de la cavité buccale, c'est-à-dire la muqueuse ou les dents.

4. La composition selon les demandes 1 à 3 se **caractérise par le fait qu'**un seul dosage de l'application contient de 00.10 à 2.00 mg, de préférence de 0.25 mg à 1.00 mg de la nicotine.
